# EUROPEAN PATENT APPLICATION

(11) **EP 4 659 655 A1**
(43) Date of publication of application: **10.12.2025**
(21) Application number: 24179625.9
(22) Date of filing: 03.06.2024
(51) Int. Cl.: A61B 5/00, A61B 5/107

(54) **A PREGNANCY MONITORING SYSTEM AND METHOD**

(71) Applicant: Koninklijke Philips N.V., 5656 AG Eindhoven (NL)
(72) Inventor: BRAHMACHARI, Aveek Shankar, Eindhoven (NL); JOCHEMS, Anne, Eindhoven (NL)
(74) Representative: Philips Intellectual Property & Standards

(57) **Abstract**

A pregnancy monitoring system comprises an abdomen inspection system for determining the external shape of the abdomen of a pregnant woman. A predetermined shape is fitted to the determined external shape by a processor, and the size and/or orientation of the fitted shape is determined.

## Description

### FIELD OF THE INVENTION

The invention relates to a system and method for monitoring the progress of pregnancy.

### BACKGROUND OF THE INVENTION

The journey of a baby in an expectant mother's womb is the most wonderful thing nature has bestowed and thus, it is important to make sure that this journey is carried out safely leading to a healthy mother and baby.

Typically, most expecting mothers pay visits to doctors on a regular basis. However, increasingly mothers want to know about the well-being of their baby on a day-to-day basis. The tests that can be done in a laboratory cannot be done at home.

One proposed solution is to wear a belt that monitors the activity of the baby. In addition to the activity of the baby, it is of interest to know the position and orientation of the baby.

CN 107397535 discloses a system for fetal heart monitoring and fetal movement detection. Fetal position can be detected by detecting the position of the legs and the position of the heart. The legs are identified as being at the location where there is the most frequent fetal movement.

It would be of interest to obtain information which can be used to track the development of the fetus.

### SUMMARY OF THE INVENTION

The invention is defined by the claims.

According to examples in accordance with an aspect of the invention, there is a pregnancy monitoring system, comprising:
an abdomen inspection system for determining the external shape of the abdomen of a pregnant woman; and
a processor configured to process the determined external shape, wherein the processor is configured to:
   fit a predetermined shape to the determined external shape; and
   determine at least one of: the size and orientation of the fitted shape.

This monitoring method assumes that the general area occupied by a fetus is a known predetermined shape, and that the external surface of the expectant mother's abdomen can be modelled as a portion of this shape. There is a scaling between the mother's abdomen and the fetus so that determining the size and/or orientation of the modelled shape can be used to track the growth and/or position of the fetus.

In this way, a baby's development can be tracked over a period.

The processor is for example configured to track the size and/or shape of the fitted shape over time. In this way, the fetus size and/or orientation is tracked using the fitted shape. The orientation of the modelled surface shape corresponds to the orientation of the fetus. Thus, the position and orientation of the baby can be derived, and the baby's development and movement can be tracked over a period.

The predetermined shape preferably comprises a three-dimensional shape, for example an ellipsoid.

The method in this example assumes that the general area occupied by a fetus is an ellipsoid, and that the surface of the expectant mother's abdomen can be modelled as a portion of an ellipsoid.

The processor is for example configured to determine an error between the predetermined shape and the determined external shape.

The position of the legs or head may for example be deduced from the modelling error of the ellipsoid. For example, a fetus with legs folded may be shaped closely to a pear, with the head at the narrower end. Hence, the ellipsoid modelling has errors that may be used to indicate the positions of the head and/or legs. In this present example, the ellipsoid (or in another situation another shape used for fitting) may be wider than the head, but narrower than the legs, resulting in fitting errors of opposite sign.

The processor is for example configured to detect changes in the fitted shape over time.

A change in shape over short time periods may be used to detect movement of the fetus. The change in shape is for example derived from the error between the ellipsoid shape and the determined IMU positions. A large error and a sudden change in shape would suggest fetal activity.

The abdomen inspection system for example comprises an abdomen belt comprising an array of inertial measurement units, IMUs for positioning against the abdomen of a pregnant woman, and the processor is configured to process the signals captured by the IMUs to:
determine positions of the IMUs; and
fit the predetermined shape to the determined positions.

This is one way to obtain the external shape information. Because motion sensors are used for the position determination in this example, the movement of the baby can also be tracked, and thus a report can be provided to indicate that everything is normal in terms of activity as well as development.

The IMUs preferably obtain position information relative to each other.

The processor is for example configured to monitor local movements at the IMUs and derive a measure of baby movement from the local movements.

Thus, local movements, rather than overall shape changes, may also be used to detect fetal movements.

The processor is for example configured to:
detect regions of greatest movement and determine an orientation of the fetus by assuming the legs are at regions of greatest movement and/or
detect regions of least movement and determine an orientation of the fetus by assuming the head is at a region of least movement.

Thus, the movement information may be used to derive fetal position information.

The processor may be configured to:
determine the orientations of the IMUs;
determine the center of the ellipsoid shape from the IMU orientations; and
refine the determined positions of the IMUs using geometric constraints.

The orientations of the IMUs can be used to find the center of the modelled ellipsoid, and this ellipsoid center can help in refining the positions and orientations of the IMUs by extremely small movements until there is no further convergence. The IMU measurements may not be perfect so can be refined with geometric constraints. The error can never be reduced to zero, but the remaining error can be used to derive movement and orientation information.

The invention also provides a pregnancy monitoring method, comprising:
receiving external shape information of the abdomen of a pregnant woman; and
processing the external shape information by:
   fitting a predetermined shape to the external shape information; and
   determining at least one of: the size and orientation of the fitted shape.

The predetermined shape for example comprises an ellipsoid.

The processor is for example configured to determine an error between the predetermined shape and the determined external shape.

The method may comprise detecting changes in the fitted shape over time. This may be used to detect movement.

In one example, the external shape information comprises inertia measurement unit signals of an array of inertial measurement units of an abdomen belt, and the method comprises:
determining positions of the IMUs; and
fitting the predetermined shape to the determined positions.

The use of IMU signals enables local movements to be monitored at the IMUs to derive a measure of baby movement. Regions of greatest movement and/or regions of least movement may be identified, and an orientation of the fetus may be obtained by assuming the legs are at regions of greatest movement and/or by assuming the head is at a region of least movement.

The method may comprise positioning the IMUs with known relative positions as part of a calibration procedure before applying the abdomen belt against the abdomen of the pregnant woman. This enables the movement monitoring to provide relative positional information between the array of IMUs.

The invention also provides a computer program comprising computer program code which is adapted, when said program is run on a computer, to implement the method defined above.

The invention also provides a processor programmed by the computer program.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an abdomen belt;
Fig. 2 shows the belt worn by an expectant mother;
Fig. 3 shows the overall pregnancy monitoring system;
Fig. 4 shows a fetus modelled as an ellipsoid;
Fig. 5 shows how the ellipsoid changes in size and orientation over time; and
Fig. 6 shows the method performed by the processor to provide the monitoring function.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a pregnancy monitoring system comprises an abdomen inspection system for determining the external shape of the abdomen of a pregnant woman. A predetermined shape is fitted to the determined external shape by a processor, and the size and/or orientation of the fitted shape is determined.

The invention will be described with reference to one detailed example, and alternative implementations will then be outlined.

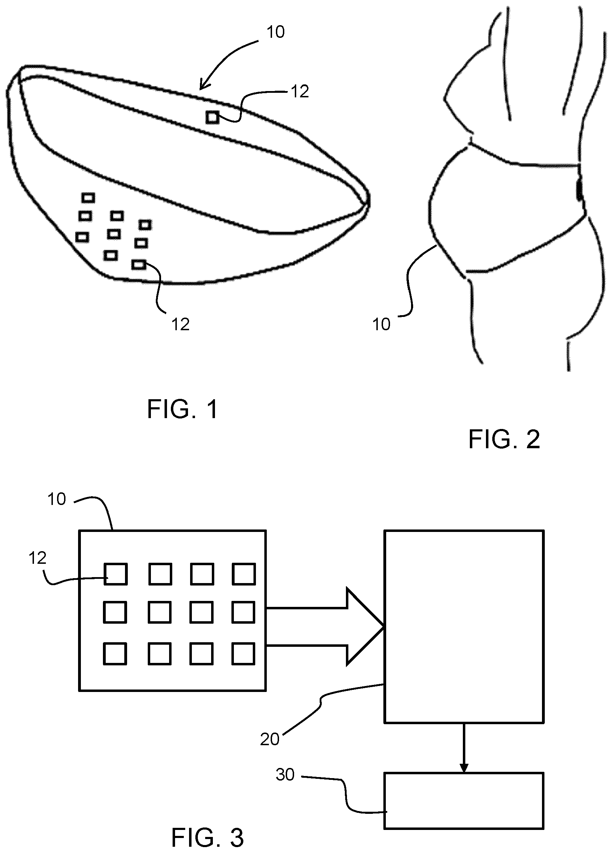

Fig. 1 shows an abdomen inspection system in the form of an abdomen belt 10 comprising an array of inertial measurement units (IMUs) 12 for positioning against the abdomen of a pregnant woman. In the example shown, there is an array of IMUs for positioning over the belly and one at the back. The IMU at the back is for example a reference IMU, and the positions of all of the front IMUs are determined relative to the one at the back. However, any IMU may be used as the reference.

The IMUs are used to determine the external shape of the abdomen, and provide external shape information for this purpose.

The IMUs each comprise at least a 3-axis accelerometer so that their position may be tracked over time (by integration). Preferably, they also comprise a 3-axis gyroscope, so that position and orientation may be tracked over time. The IMUs also preferably include a magnetometer.

Fig. 2 shows the belt 10 worn by an expectant mother. The array of IMUs sits against the belly of the mother and thus the belt adopts the shape of the belly. It is flexible and stretchable to maintain contact with the belly while being comfortable to wear.

Fig. 3 shows the overall pregnancy monitoring system, comprising the belt 10, a processor 20 and an output interface 30. The output interface provides advice to the expectant mother. It may for example be a portable wireless device of the expectant mother (mobile phone or tablet) with which the processor 20 communicates over a wireless interface.

The processor 20 is configured to process the signals captured by the IMUs 12 (and more generally to process external shape information from the abdomen inspection system).

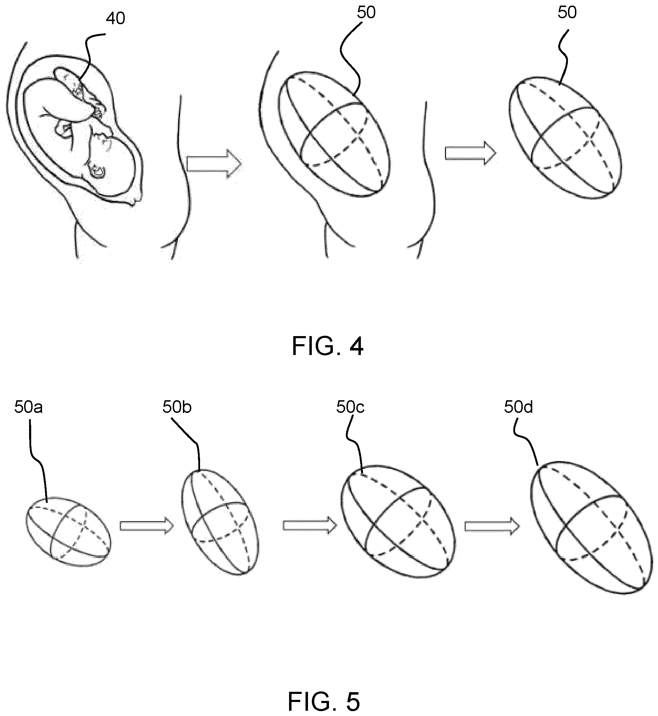

Fig. 4 shows a fetus 40. The fetus is modelled as a predetermined shape, preferably a three-dimensional shape, which in this example is an ellipsoid 50. The size and/or orientation of that ellipsoid is monitored over time, by monitoring the shape of the abdomen of the expectant mother using the belt 10, which reports the positions of the IMUs relative to each other.

Fig. 5 shows how the ellipsoid changes in size and orientation over time (between size/orientations 50a, 50b, 50c and 50d).

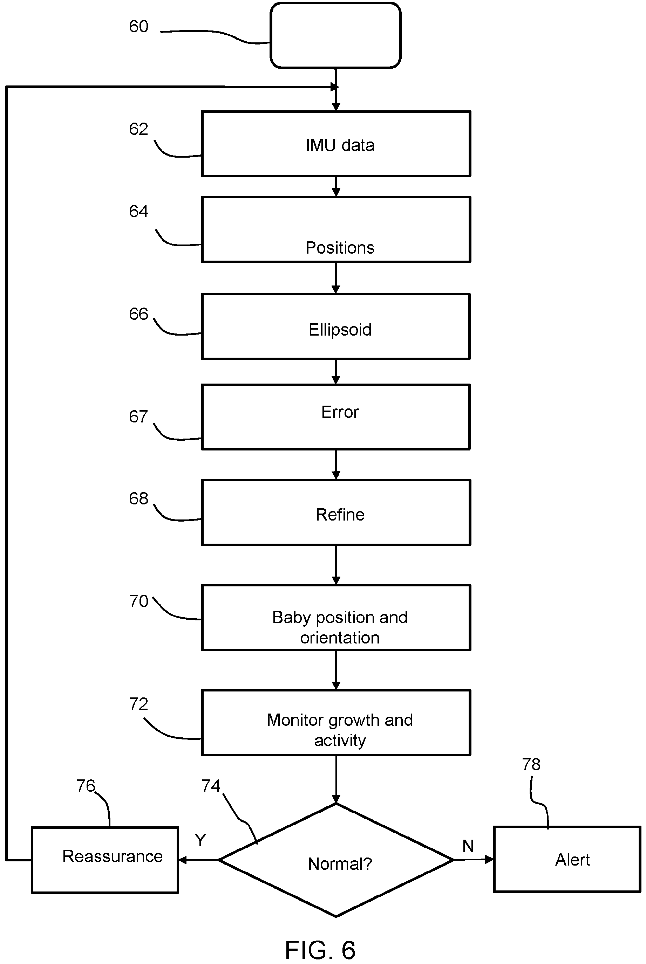

Fig. 6 shows the method performed by the processor to provide the monitoring function.

The process starts in step 60.

In step 62, streaming data from the IMUs 12 is captured by the processor 20.

In step 64, the positions of the IMUs are obtained relative to each other, for example relative to a reference IMU. For this purpose, a calibration process may have been carried out, by which the belt is applied to a template so that the relative positions of the IMUs are known. Movement of the IMUs can then be tracked by known methods, by cancelling the gravity vectors and integrating the acceleration vectors. By re-calibrating each time the belt is to be worn, signal drift can be reduced.

More generally, steps 62 and 64 involve receiving external shape information of the abdomen.

The IMU sensors report orientation information. The readings of the multiple sensors can be stabilized using sensor fusion by means of a Kalman filter. Before Kalman filtering, low pass filtering is performed to remove noise. Such sensor fusion approaches are for example well known for flying swarms of drones for air show purposes to avoid the need for frequent recalibration. The present application enables the use of additional geometric constraints such as the size of the belly or fetus, as these cannot change significantly over a single day, hence restricting the drift error.

In step 66, an ellipsoid shape is fitted to the determined positions. More generally, a predetermined shape is fitted to the external shape information.

This is based on the assumption that the general area occupied by the fetus is a known shape, such as an ellipsoid, and that the surface of the expectant mother's abdomen can be modelled as part of an ellipsoid. There is of course a scaling between the mother's abdomen and the fetus so that the fitted ellipsoid is not the same size as the fetus. However, tracking changes in the the size of the modelled ellipsoid can be used to track the growth of the fetus without any need for the scaling factor. Furthermore, a scaling factor can, if desired, be assumed between the abdomen ellipsoid size and the fetus size, and this scaling factor may take account of the expectant mother's physiology, such as the amount of subcutaneous fatty tissue, which may be indicated by a BMI measurement. A direct linear mapping of the fetus shape and size to the abdomen shape and size may be assumed, only differing in scale.

The baby's development can be tracked over a period. Because motion sensors are used for the position determination in this example, the movement of the baby (that is detectable at the abdomen surface) can also be tracked.

In a most basic implementation, the size of the fitted ellipsoid shape can then be tracked over time (in step 72). However, some additional optional processing steps will also be described.

In step 67, an error between the ellipsoid shape and the determined IMU positions is determined, i.e., a fitting error. The fitting error can be aggregated for all IMUs. This gives an indication of how far the abdomen shape deviates from an ellipsoid. This information can have various uses. The position of the legs or head may for example be deduced from the modelling error of the ellipsoid. For example, a fetus with legs folded is shaped like a pear, with the head at the narrower end. Hence, the ellipsoid modelling has errors that may be used to indicate the positions of the head and/or legs.

The sign of the error between the fitted (ellipsoid) shape and the actual shape of the abdomen may also be used to indicate the orientation of the fetus. A fetus may for example be pear-shaped with the wider area indicating the lower half of the body. Thus, the fitting error may be of one sign (the fitted shape is too small) in the area of the lower body (the legs of the fetus) whereas the fitting error may be of the opposite sign (the fitted shape is too large) in the area of the upper body (the head of the fetus).

In optional step 68, corrections may be made for errors in the IMU position information caused by signal drift. The orientations of the IMUs (using gyroscope sensors of the IMUs) can also help to find the center of the ellipsoid (for example where the normal axes of the IMUs meet or are closest to meeting). This center of the ellipsoid can help in refining the positions and orientations of the IMUs.

Thus, the IMU measurements can be refined using geometric constraints. as mentioned above, to reduce a fitting error. This refinement of the IMU data, by which sensor measurements are adjusted to provide correction of drift, can avoid the need for frequent recalibration of the belt.

One further approach is to use the Kalman filtered orientation to produce nearby points (arranged in a small square or circle around the center of the IMU). These points, together with the centers, give a better estimate of the ellipsoid. There may also be more points around a reference IMU, such as the IMU at the back of the belt.

In step 70, the orientation of the fetus is determined as well as the baby position (e.g., head up or down). The orientation as well as the size of the fitted ellipsoid shape can be monitored over time as shown in Figure 5.

In addition, changes in the ellipsoid shape can be monitored over time using the fitting error obtained in step 67. A change in shape over short time periods may be used to detect movement of the fetus. A large error and a sudden change in shape would suggest fetal activity.

The orientation of the baby may include the fetus orientation (obtained from the major and minor axes of the fitted ellipsoid) but also the orientation of the baby within the fetus area (i.e. head up or down). For example, regions of greatest (local) movement may correspond to the legs and regions of least movement may correspond to the head. Local movements at the IMUs may also be used to derive a measure of the overall baby movement i.e., activity. Thus, local movements, rather than overall shape changes or baby orientation changes, may also be used to detect fetal movements.

In step 72 the movement and orientation information over time as well as the size information is processed to provide a measure of growth and activity level.

In step 74 it is determined if the monitored growth and activity is normal. If not, an alert is provided in step 78 (via the user interface 30). If everything is normal, reassurance is provided in step 76 (again via the user interface 30) and the monitoring continues.

In addition to movement and shape of the fetus, the IMUs may be used to monitor the expectant mother's breathing rate, and movement frequencies at the breathing rate may be filtered out of the IMU signals.

The IMU data may be processed with normalization and non-maximal suppression. Regions of fetal movement may be localized and patterns in position and magnitude may be analyzed to narrow down to possible fetal movements.

The activity may be presented as a movement percentage, and this may be provided as a report when triggered by the expectant mother.

The example above uses IMUs to derive position information. Another example of an abdomen inspection system is a structured light system which projects a light-based calibration grid of a known shape with points at known distances on the abdomen. Image sensing is used to estimate the position of the points, including depth of the points from the camera. Recovering the points from the images and matching them with the reference grid enables the external shape to be derived.

The ellipsoid is one example of a suitable predetermined shape. A more complex shape can be derived as a combination of other shapes, such as multiple ellipsoids, for example. Fitting to two ellipsoids may for example enable separated detection of the head and body areas of the abdomen. More generally, the predetermined shape may be a 2D shape, or a 3D shape, or a stack of 2D shapes, for example.

As explained above, the processor of the system processes the external abdomen shape. This external shape may be received from the abdomen monitoring system, in which case the abdomen monitoring system has its own processor that derives the external shape from the raw sensor data. However, in another example, the abdomen monitoring system may provide raw sensor data to the processor of the monitoring system, and that processor then first derives the external shape before then processing that shape by fitting the predetermined shape to it.

In all cases, the determined external 3D shape may be reconstructed from a set of measured 2D slices, and the fitting may also involve fitting 2D slices.

The invention may be used for tracking development and movement over time, but it may be used for individual measurements of size (for example if a scaling between the abdomen size and fetus size has previously been determined) or orientation.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A pregnancy monitoring system, comprising:
an abdomen inspection system for determining the external shape of the abdomen of a pregnant woman; and
a processor (20) configured to process the determined external shape, wherein the processor is configured to:
(66) fit a predetermined shape to the determined external shape; and
(72) determine at least one of: the size and orientation of the fitted shape.

2. The system of claim 1, wherein the processor is configured to track the at least one of size and shape of the fitted shape over time.

3. The system of claim 1 or 2, wherein the predetermined shape comprises an ellipsoid.

4. The system of any one of claims 1 to 3, wherein the processor is configured to:
(67) determine an error between the predetermined shape shape and the determined external shape.

5. The system of any one of claims 1 to 4, wherein the processor is configured to:
detect changes in the fitted shape over time.

6. The system of any one of claims 1 to 5, wherein:
the abdomen inspection system comprises an abdomen belt (10) comprising an array of inertial measurement units (12), IMUs, for positioning against the abdomen of a pregnant woman; and
the processor (20) is configured to process the signals captured by the IMUs to: (64) determine positions of the IMUs; and
(66) fit the predetermined shape to the determined positions.

7. The system of claim 6, wherein the processor is configured to:
monitor local movements at the IMUs and derive a measure of baby movement from the local movements.

8. The system of claim 7, wherein the processor is configured to perform at least one of the following actions:
detect regions of greatest movement and determine an orientation of the fetus by assuming the legs are at regions of greatest movement and
detect regions of least movement and determine an orientation of the fetus by assuming the head is at a region of least movement.

9. A pregnancy monitoring method, comprising:
(60) receiving external shape information of the abdomen of a pregnant woman; and processing the external shape information by:
(66) fitting a predetermined shape to the external shape information; and
(72) determining at least one of: the size and orientation of the fitted shape.

10. The method of claim 9, wherein the predetermined shape comprises an ellipsoid.

11. The method of claim 9 or 10, wherein the processor is configured to:
(67) determine an error between the predetermined shape and the external shape information.

12. The method of any one of claims 9 to 11, comprising detecting changes in the fitted shape over time.

13. The method of any one of claims 9 to 12, wherein:
the external shape information comprises inertia measurement unit signals of an array of inertial measurement units (12) of an abdomen belt (10), and the method comprises:
(64) determining positions of the IMUs relative to each other; and
(66) fitting the predetermined shape to the determined positions.

14. A computer program comprising computer program code which is adapted, when said program is run on a computer, to implement the method of any one of claims 9 to 13.

15. A processor which is programmed with the computer program of claim 14.
